# EUROPEAN PATENT SPECIFICATION

(11) **EP 0 865 465 B1**
(45) Date of publication and mention of the grant of the patent: **30.05.2001**
(21) Application number: 96938989.9
(22) Date of filing: 29.11.1996
(51) Int. Cl.: C09B 67/00, A61K 7/13

(54) **LACCASES WITH IMPROVED DYEING PROPERTIES**
LACCASEN MIT VERBESSERTEN FÄRBEEIGENSCHAFTEN
LACCASE AVEC DES PROPRIETES COLORANTES AMELIOREES

(30) Priority: 30.11.1995 DK 135795
(43) Date of publication of application: 23.09.1998
(73) Proprietor: Novozymes A/S, 2880 Bagsvaerd (DK)
(72) Inventor: AASLYNG, Dorrit, DK-2880 Bagsvaerd (DK); SOERENSEN, Niels Henrik, DK-2880 Bagsvaerd (DK); ROERBAEK, Karen, DK-2880 Bagsvaerd (DK)
(86) International application number: DK9600499
(87) International publication number: WO9719999

(56) References cited:
- EP-A- 0 504 005
- WO-A-94/00100
- WO-A-95/07988
- WO-A-95/33836
- WO-A-95/33837
- WO-A-96/00290
- DE-A- 4 314 317
- US-A- 3 251 742
- STN INTERNATIONAL, File CAPLUS, CAPLUS Accession No. 1991:498981, SARUNO, RINJIRO: "Hair-Dyeing Preparations Containing Melanin or Other Polyphenol Pigments and Manufacture of the Pigments"; & JP,A,03 077 813, (03-04-91).
- STN INTERNATIONAL, File CAPLUS, CAPLUS Accession No. 1995:974547, CHUVUKULA, MURALIKRISHNA et al., "Phenolic Azo Dye Oxidation by Laccase From Pyricularia Oryzae"; & APPL. ENVIRON. MICROBIOL. (1995), 61(12), 4374-77.

### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (D) COUNTRY: Denmark
      (E) POSTAL CODE (ZIP): DK-2880
      (F) TELEPHONE: +45 4444 8888
      (G) TELEFAX: +45 4449 3256
   (ii) TITLE OF INVENTION:
      Laccases with improved dyeing properties
   (iii) NUMBER OF SEQUENCES: 2
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
(2) INFORMATION FOR SEQ ID NO:1:
   (i) SEQUENCE CHARACTERISTICS:
      (A) LENGTH: 3192 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: join(586..831, 917..994, 1079..1090, 1193..1264, 1337..2308, 2456..2524, 2618..3028)
   (xi) SEQUENCE DESCRIPTION: SEQ ID NO:1:
   TCTAGCTTCT TTGGTCACCG TCGTTTTCGC CCGCCCCCTC CCTCCTTCAA CCCCCTGAGT       60
   AGTCGGCTAA GCGATCCTCA ATCTGGTCTT GTGAGGTCAC GTCCTCCAGC AGATGACAGT      120
   TCATCGAGCG AGTGATCTCC ACCACCCAGA AGGGAGGGGG GATGCGCGCA TGCTCCAACA      180
   TCCCTGGTGT CGCTAGAGAC GTCGCGGCAT CAGCCTTTTC ATCACACCGA GCACGTCCAC      240
   GGACCGGCTC CTTTCACCCC CGCGTCCTCC GGAGGATTGA GTCACGATAT TTCGGGATGT      300
   GGGAAGGGGG AGAGAAAGGA GGGGGGAGGG GCGGAAACAT GTTGGATACG AGCTGCGCCC      360
   CTTTTTCAAC ATCGAGAACA GGAAGTCGTT GGTGTCGGCC GTAATGTCTA TAAAACGAGG      420
   CTCCTTCTCG TCGTCGACTT GTCTCAGGTT CTCTCTCTCG TCCACACCAA GCCAGTCTTG      480
   CCTGAGCCAC CTGAGCCACC TTCAACTCAT CATCTTCAGT CAAGTCGTTC ATTGACATTG      540
   TGTCTCTCTT TCTATCGAGT CGGCTTCCCG GCCCTTCACC ACAAC ATG AAG TCC          594
                                                     Met Lys Ser
                                                       1
   TTC ATC AGC GCC GCG ACG CTT TTG GTG GGC ATT CTC ACC CCT AGC GTT        642
   Phe Ile Ser Ala Ala Thr Leu Leu Val Gly Ile Leu Thr Pro Ser Val
        5                  10                  15
   GCT GCT GCC CCT CCA TCC ACC CCT GAG CAG CGC GAC CTG CTC GTC CCG      690
   Ala Ala Ala Pro Pro Ser Thr Pro Glu Gln Arg Asp Leu Leu Val Pro
    20                 25                  30                  35
   ATC ACG GAG AGG GAG GAG GCA GCC GTG AAG GCT CGC CAG CAG AGC TGC      738
   Ile Thr Glu Arg Glu Glu Ala Ala Val Lys Ala Arg Gln Gln Ser Cys
                   40                  45                  50
   AAC ACC CCC AGC AAC CGG GCG TGC TGG ACT GAC GGA TAC GAC ATC AAC      786
   Asn Thr Pro Ser Asn Arg Ala Cys Trp Thr Asp Gly Tyr Asp Ile Asn
               55                  60                  65
   ACC GAC TAC GAA GTG GAC AGC CCG GAC ACG GGT GTT GTT CGG CCG          831
   Thr Asp Tyr Glu Val Asp Ser Pro Asp Thr Gly Val Val Arg Pro
           70                  75                  80
   GTGAGTGCTC TCGTTAATTA CGCTTCGGCG AGTTGCGCAG ATATATTAAA TACTGCAAAC    891
   CTAAGCAGGA GCTGACATGC GACAG TAC ACT CTG ACT CTC ACC GAA GTC GAC      943
                               Tyr Thr Leu Thr Leu Thr Glu Val Asp
                                       85                  90
   AAC TGG ACC GGA CCT GAT GGC GTC GTC AAG GAG AAG GTC ATG CTG GTT      991
   Asn Trp Thr Gly Pro Asp Gly Val Val Lys Glu Lys Val Met Leu Val
               95                 100                 105
   AAC GTACGGCACC CCTTTTCTTG TCCTAGGATC TGGGTGATGT GCGTCGTTGC          1044
   Asn
   CCCTGAGAGA GACTGACCGA GCCTTTGGCT GCAG AAT AGT ATA ATC GTAATTAATT    1100
                                         Asn Ser Ile Ile
                                             110
   ATACCGCCCT GCCTCCAGCA GCCCCAGCAG CTCGAGAAGG GTATCTGAAG TTAGTCAGGC   1160
   CTGCTGACCT GACCGGGGCC AACCCACCAT AG GGA CCA ACA ATC TTT GCG GAC      1213
                                       Gly Pro Thr Ile Phe Ala Asp
                                               115
   TGG GGC GAC ACG ATC CAG GTA ACG GTC ATC AAC AAC CTC GAG ACC AAC      1261
   Trp Gly Asp Thr Ile Gln Val Thr Val Ile Asn Asn Leu Glu Thr Asn
   120                 125                 130                 135
   GGC GTATGTCTGC TGCTTGCTCT CTTGCTCTCC TCGTCCGCGA CTAATAATAA          1314
   Gly
   TATCAACTCG TGTGGAAAAC AG ACG TCG ATC CAC TGG CAC GGA CTG CAC CAG     1366
                            Thr Ser Ile His Trp His Gly Leu His Gln
                                        140                 145
   AAG GGC ACC AAC CTG CAC GAC GGC GCC AAC GGT ATC ACC GAG TGC CCG      1414
   Lys Gly Thr Asn Leu His Asp Gly Ala Asn Gly Ile Thr Glu Cys Pro
               150                 155                 160
   ATC CCG CCC AAG GGA GGG AGG AAG GTG TAC CGG TTC AAG GCT CAG CAG      1462
   Ile Pro Pro Lys Gly Gly Arg Lys Val Tyr Arg Phe Lys Ala Gln Gln
           165                 170                 175
   TAC GGG ACG AGC TGG TAC CAC TCG CAC TTC TCG GCC CAG TAC GGC AAC      1510
   Tyr Gly Thr Ser Trp Tyr His Ser His Phe Ser Ala Gln Tyr Gly Asn
       180                 185                 190
   GGC GTG GTC GGG GCC ATT CAG ATC AAC GGG CCG GCC TCG CTG CCG TAC      1558
   Gly Val Val Gly Ala Ile Gln Ile Asn Gly Pro Ala Ser Leu Pro Tyr
   195                 200                 205                 210
   GAC ACC GAC CTG GGC GTG TTC CCC ATC AGC GAC TAC TAC TAC AGC TCG      1606
   Asp Thr Asp Leu Gly Val Phe Pro Ile Ser Asp Tyr Tyr Tyr Ser Ser
                   215                 220                 225
   GCC GAC GAG CTG GTG GAA CTC ACC AAG AAC TCG GGC GCG CCC TTC AGC      1654
   Ala Asp Glu Leu Val Glu Leu Thr Lys Asn Ser Gly Ala Pro Phe Ser
               230                 235                 240
   GAC AAC GTC CTG TTC AAC GGC ACG GCC AAG CAC CCG GAG ACG GGC GAG      1702
   Asp Asn Val Leu Phe Asn Gly Thr Ala Lys His Pro Glu Thr Gly Glu
           245                 250                 255
   GGC GAG TAC GCC AAC GTG ACG CTC ACC CCG GGC CGG CGG CAC CGC CTG      1750
   Gly Glu Tyr Ala Asn Val Thr Leu Thr Pro Gly Arg Arg His Arg Leu
       260                 265                 270
   CGC CTG ATC AAC ACG TCG GTC GAG AAC CAC TTC CAG GTC TCG CTC GTC      1798
   Arg Leu Ile Asn Thr Ser Val Glu Asn His Phe Gln Val Ser Leu Val
   275                 280                 285                 290
   AAC CAC ACC ATG ACC ATC ATC GCC GCC GAC ATG GTG CCC GTC AAC GCC      1846
   Asn His Thr Met Thr Ile Ile Ala Ala Asp Met Val Pro Val Asn Ala
                   295                 300                 305
   ATG ACG GTC GAC AGC CTC TTC CTC GGC GTC GGC CAG CGC TAC GAT GTC      1894
   Met Thr Val Asp Ser Leu Phe Leu Gly Val Gly Gln Arg Tyr Asp Val
               310                 315                 320
   GTC ATC GAA GCC AGC CGA ACG CCC GGG AAC TAC TGG TTT AAC GTC ACA      1942
   Val Ile Glu Ala Ser Arg Thr Pro Gly Asn Tyr Trp Phe Asn Val Thr
           325                 330                 335
   TTT GGC GGC GGC CTG CTC TGC GGC GGC TCC AGG AAT CCC TAC CCG GCC      1990
   Phe Gly Gly Gly Leu Leu Cys Gly Gly Ser Arg Asn Pro Tyr Pro Ala
       340                 345                 350
   GCC ATC TTC CAC TAC GCC GGC GCC CCC GGC GGC CCG CCC ACG GAC GAG      2038
   Ala Ile Phe His Tyr Ala Gly Ala Pro Gly Gly Pro Pro Thr Asp Glu
   355                 360                 365                 370
   GGC AAG GCC CCG GTC GAC CAC AAC TGC CTG GAC CTC CCC AAC CTC AAG      2086
   Gly Lys Ala Pro Val Asp His Asn Cys Leu Asp Leu Pro Asn Leu Lys
                   375                 380                 385
   CCC GTC GTG GCC CGC GAC GTG CCC CTG AGC GGC TTC GCC AAG CGG CCC      2134
   Pro Val Val Ala Arg Asp Val Pro Leu Ser Gly Phe Ala Lys Arg Pro
               390                 395                 400
   GAC AAC ACG CTC GAC GTC ACC CTC GAC ACC ACG GGC ACG CCC CTG TTC      2182
   Asp Asn Thr Leu Asp Val Thr Leu Asp Thr Thr Gly Thr Pro Leu Phe
           405                 410                 415
   GTC TGG AAG GTC AAC GGC AGC GCC ATC AAC ATC GAC TGG GGC AGG CCC      2230
   Val Trp Lys Val Asn Gly Ser Ala Ile Asn Ile Asp Trp Gly Arg Pro
       420                 425                 430
   GTC GTC GAC TAC GTC CTC ACG CAG AAC ACC AGC TTC CCA CCC GGG TAC      2278
   Val Val Asp Tyr Val Leu Thr Gln Asn Thr Ser Phe Pro Pro Gly Tyr
   435                 440                 445                 450
   AAC ATT GTC GAG GTG AAC GGA GCT GAT CAG GTAAGAAAAA GGGGACCGCA        2328
   Asn Ile Val Glu Val Asn Gly Ala Asp Gln
                   455                 460
   GGGGTGCTGC TGCAAGTACA CCTTGCTCGC CCTCCTGTTC TTCCTTAATA ACTACCTCCC    2388
   AACCCTCCCC CCTAATTAAT TCACTTTAAA GGCCGATCAA GACTGACCGA GCCCCCTCTC    2448
   TTTGCAG TGG TCG TAC TGG TTG ATC GAG AAC GAT CCC GGC GCA CCT TTC      2497
           Trp Ser Tyr Trp Leu Ile Glu Asn Asp Pro Gly Ala Pro Phe
                                   465                 470
   ACC CTA CCG CAT CCG ATG CAC CTG CAC GTAAGTTGGA TACATATATA          2544
   Thr Leu Pro His Pro Met His Leu His
   475                 480
   TATATATATA TACATTGCTT TCCTGGCTCG CTCCCTTAAA TAAAATTAAA TAACCAAAAA   2604
   TAACAAAAAA AAG GGC CAC GAC TTT TAC GTG CTG GGC CGC TCG CCC GAC      2653
                  Gly His Asp Phe Tyr Val Leu Gly Arg Ser Pro Asp
                      485                 490                 495
   GAG TCG CCG GCA TCC AAC GAG CGG CAC GTG TTC GAT CCG GCG CGG GAC     2701
   Glu Ser Pro Ala Ser Asn Glu Arg His Val Phe Asp Pro Ala Arg Asp
                   500                 505                 510
   GCG GGC CTG CTG AGC GGG GCC AAC CCT GTG CGG CGG GAC GTG ACG ATG     2749
   Ala Gly Leu Leu Ser Gly Ala Asn Pro Val Arg Arg Asp Val Thr Met
               515                 520                 525
   CTG CCG GCG TTC GGG TGG GTG GTG CTG GCC TTC CGG GCC GAC AAC CCG     2797
   Leu Pro Ala Phe Gly Trp Val Val Leu Ala Phe Arg Ala Asp Asn Pro
           530                 535                 540
   GGC GCC TGG CTG TTC CAC TGC CAC ATC GCC TGG CAC GTC TCG GGC GGC     2845
   Gly Ala Trp Leu Phe His Cys His Ile Ala Trp His Val Ser Gly Gly
       545                 550                 555
   CTG GGC GTC GTC TAC CTC GAG CGC GCC GAC GAC CTG CGC GGG GCC GTC     2893
   Leu Gly Val Val Tyr Leu Glu Arg Ala Asp Asp Leu Arg Gly Ala Val
   560                 565                 570                 575
   TCG GAC GCC GAC GCC GAC GAC CTC GAC CGC CTC TGC GCC GAC TGG CGC     2941
   Ser Asp Ala Asp Ala Asp Asp Leu Asp Arg Leu Cys Ala Asp Trp Arg
                   580                 585                 590
   CGC TAC TGG CCT ACC AAC CCC TAC CCC AAG TCC GAC TCG GGC CTC AAG     2989
   Arg Tyr Trp Pro Thr Asn Pro Tyr Pro Lys Ser Asp Ser Gly Leu Lys
               595                 600                 605
   CAC CGC TGG GTC GAG GAG GGC GAG TGG CTG GTC AAG GCG TGAGCGAAGG      3038
   His Arg Trp Val Glu Glu Gly Glu Trp Leu Val Lys Ala
           610                 615                 620
   AGGAAAAAGG AAACAAAGAG GGGGGGGGGG GCTAGTTCCT ATTTTTGCTT TTTTTTTTTG   3098
   TTCTTGTCCT TGTGCTGGCG GTTACCCTGG TAAAGGAGAA GGGGGCCCCA AGTTCGAGTG   3158
   GGTGTGTGAT CGGGTAAATA TTATCAAGAG ATCT                               3192
(2) INFORMATION FOR SEQ ID NO:2:
   (i) SEQUENCE CHARACTERISTICS:
      (A) LENGTH: 620 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Ser Phe Ile Ser Ala Ala Thr Leu Leu Val Gly Ile Leu Thr
    1               5                  10                  15
   Pro Ser Val Ala Ala Ala Pro Pro Ser Thr Pro Glu Gln Arg Asp Leu
               20                  25                  30
   Leu Val Pro Ile Thr Glu Arg Glu Glu Ala Ala Val Lys Ala Arg Gln
           35                  40                  45
   Gln Ser Cys Asn Thr Pro Ser Asn Arg Ala Cys Trp Thr Asp Gly Tyr
       50                  55                  60
   Asp Ile Asn Thr Asp Tyr Glu Val Asp Ser Pro Asp Thr Gly Val Val
   65                  70                  75                  80
   Arg Pro Tyr Thr Leu Thr Leu Thr Glu Val Asp Asn Trp Thr Gly Pro
                   85                  90                  95
   Asp Gly Val Val Lys Glu Lys Val Met Leu Val Asn Asn Ser Ile Ile
               100                 105                 110
   Gly Pro Thr Ile Phe Ala Asp Trp Gly Asp Thr Ile Gln Val Thr Val
           115                 120                 125
   Ile Asn Asn Leu Glu Thr Asn Gly Thr Ser Ile His Trp His Gly Leu
       130                 135                 140
   His Gln Lys Gly Thr Asn Leu His Asp Gly Ala Asn Gly Ile Thr Glu
   145                 150                 155                 160
   Cys Pro Ile Pro Pro Lys Gly Gly Arg Lys Val Tyr Arg Phe Lys Ala
                   165                 170                 175
   Gln Gln Tyr Gly Thr Ser Trp Tyr His Ser His Phe Ser Ala Gln Tyr
               180                 185                 190
   Gly Asn Gly Val Val Gly Ala Ile Gln Ile Asn Gly Pro Ala Ser Leu
           195                 200                 205
   Pro Tyr Asp Thr Asp Leu Gly Val Phe Pro Ile Ser Asp Tyr Tyr Tyr
       210                 215                 220
   Ser Ser Ala Asp Glu Leu Val Glu Leu Thr Lys Asn Ser Gly Ala Pro
   225                 230                 235                 240
   Phe Ser Asp Asn Val Leu Phe Asn Gly Thr Ala Lys His Pro Glu Thr
                   245                 250                 255
   Gly Glu Gly Glu Tyr Ala Asn Val Thr Leu Thr Pro Gly Arg Arg His
               260                 265                 270
   Arg Leu Arg Leu Ile Asn Thr Ser Val Glu Asn His Phe Gln Val Ser
           275                 280                 285
   Leu Val Asn His Thr Met Thr Ile Ile Ala Ala Asp Met Val Pro Val
       290                 295                 300
   Asn Ala Met Thr Val Asp Ser Leu Phe Leu Gly Val Gly Gln Arg Tyr
   305                 310                 315                 320
   Asp Val Val Ile Glu Ala Ser Arg Thr Pro Gly Asn Tyr Trp Phe Asn
                   325                 330                 335
   Val Thr Phe Gly Gly Gly Leu Leu Cys Gly Gly Ser Arg Asn Pro Tyr
               340                 345                 350
   Pro Ala Ala Ile Phe His Tyr Ala Gly Ala Pro Gly Gly Pro Pro Thr
           355                 360                 365
   Asp Glu Gly Lys Ala Pro Val Asp His Asn Cys Leu Asp Leu Pro Asn
       370                 375                 380
   Leu Lys Pro Val Val Ala Arg Asp Val Pro Leu Ser Gly Phe Ala Lys
   385                 390                 395                 400
   Arg Pro Asp Asn Thr Leu Asp Val Thr Leu Asp Thr Thr Gly Thr Pro
                   405                 410                 415
   Leu Phe Val Trp Lys Val Asn Gly Ser Ala Ile Asn Ile Asp Trp Gly
               420                 425                 430
   Arg Pro Val Val Asp Tyr Val Leu Thr Gln Asn Thr Ser Phe Pro Pro
           435                 440                 445
   Gly Tyr Asn Ile Val Glu Val Asn Gly Ala Asp Gln Trp Ser Tyr Trp
       450                 455                 460
   Leu Ile Glu Asn Asp Pro Gly Ala Pro Phe Thr Leu Pro His Pro Met
   465                 470                 475                 480
   His Leu His Gly His Asp Phe Tyr Val Leu Gly Arg Ser Pro Asp Glu
                   485                 490                 495
   Ser Pro Ala Ser Asn Glu Arg His Val Phe Asp Pro Ala Arg Asp Ala
               500                 505                 510
   Gly Leu Leu Ser Gly Ala Asn Pro Val Arg Arg Asp Val Thr Met Leu
           515                 520                 525
   Pro Ala Phe Gly Trp Val Val Leu Ala Phe Arg Ala Asp Asn Pro Gly
       530                 535                 540
   Ala Trp Leu Phe His Cys His Ile Ala Trp His Val Ser Gly Gly Leu
   545                 550                 555                 560
   Gly Val Val Tyr Leu Glu Arg Ala Asp Asp Leu Arg Gly Ala Val Ser
                   565                 570                 575
   Asp Ala Asp Ala Asp Asp Leu Asp Arg Leu Cys Ala Asp Trp Arg Arg
               580                 585                 590
   Tyr Trp Pro Thr Asn Pro Tyr Pro Lys Ser Asp Ser Gly Leu Lys His
           595                 600                 605
   Arg Trp Val Glu Glu Gly Glu Trp Leu Val Lys Ala
       610                 615                 620

## Description

### FIELD OF THE INVENTION

The present invention relates to a dyeing composition comprising a microbial laccase, the use of said dyeing composition for dyeing keratinous fibres, in particular hair, fur, hide and wool, and a method for dyeing keratinous fibres.

### BACKGROUND OF THE INVENTION

It has been used for many years to dye the hair to cover appearing grey hair. The need to do so arises from the fact that grey hair is the first sign of having past adolescence, which can be hard to accept for many people.

For instance, in certain parts of Asia it is widely used by both men and women to dye the hair with dyes often referred to by humorous people as "black shoe polish".

During the last few decades hair dyeing has become more and more popular in the western world. At first Punk Rockers and other society critical groups dyed their hair in extreme colours as a part of their protest against the established society, but today especially many young people also uses hair dyes (in more soft tints than the Punk Rockers) as a sort of "cosmetic" to change or freshen up their "look".

### Hair dyes

In general hair dyeing compositions on the market today can be divided into three main groups:
- temporary hair dyes,
- semi-permanent hair dyes, and
- permanent oxidative hair dyes.

The temporary hair dyes are only intended to change the natural hair colour for a short period of time and usually functions by depositing dyes on the surface of the hair. Such hair dyes are easy to remove with normal shampooing.

When using semi-permanent hair dyes the colour of the dyed hair can survive for five or more shampooings. This is achieved by using dyes having a high affinity for hair keratin and which is able penetrate into the interior of the hair shaft.

Permanent hair dyes are very durable to sunlight, shampooing and other hair treatments and need only to be refreshed once a month as new hair grows out. With these dyeing systems the dyes are created directly in and on the hair. Small aromatic colourless dye precursors (*e.g.* p-phenylene-diamine and o-aminophenol) penetrate deep into the hair where said dye precursors are oxidised by an oxidising agent into coloured polymeric compounds. These coloured compounds are larger than the dye precursors and can not be washed out of the hair.

By including compounds referred to as modifiers (or couplers) in the hair dyeing composition a number of hair colour tints can be obtained. Cathecol and Resorcinol are examples of such modifiers.

Traditionally H₂O₂ is used as the oxidizing agent (colour builder), but also as a bleaching agent. Dyeing compositions comprising H₂O₂ are often referred to as "lightening dyes" due to this lightening effect of H₂O₂.

The use of H₂O₂ in dyeing compositions have some disadvantages as H₂O₂ damages the hair. Further, oxidative dyeing often demands high pH (normally around pH 9-10), which also inflicts damage on the hair and on the skin. Consequently, if using dye compositions comprising H₂O₂ it is not recommendable to dye the hair often. To overcome the disadvantages of using H₂O₂ it has been suggested to use oxidation enzymes to replace H₂O₂.

US patent no. 3,251,742 (Revlon) describes a method for dyeing human hair by dye formation *in situ* (*i.e.* on the hair). An oxidative enzyme is used to the colour formation reactions at a substantially neutral pH (7-8.5). Laccases, tyrosinases, polyphenolases and catacolases are mentioned as suitable oxidation enzymes. The only exemplified oxidation enzyme is tyrosinase.

EP patent no. 504.005 (Perma S.A.) concerns dyeing compositions for keratinous fibres, in particular hair, which do not require the presence of H₂O₂ (hydrogen peroxide). The composition comprises an enzyme capable of catalysing the formation of the polymeric dyes and also dye precursors, such as bases and couplers, in a buffer solution wherein the pH of said composition is between 6.5 and 8 and said enzyme has an optimal activity in the pH range between 6.5 and 8.

*Rhizoctonia praticola* laccase and *Rhus vernicifera* laccase are the only laccases exemplified in the patent.

Abstract of Papers American Chemical Society vol. 209, no. 1-2, 1995 discloses the cloning of laccases from *Scytalidium thermophilum* and *Myceliophthora thermophila.* The abstract does not mention the use of said laccases for dyeing.

### SUMMARY OF THE INVENTION

The object of the present invention is to provide improved dyeing compositions for keratinous fibres, such as hair, fur hide and wool, comprising an oxidative enzyme as the oxidising agent.

In the context of the present invention an "improved" composition for dyeing keratinous fibres means a composition being capable of dyeing the keratinous fibres in question faster or by the use of a smaller amount of oxidation enzyme to obtain an optimal dyeing effect, determined as ΔE*, in comparison to corresponding prior art dyeing compositions.

Further, it is also possible to use a less amount of dye precursor. This is advantageous as certain dye precursors are very unhealthy and very carcinogenic.

Compositions capable of dyeing the keratinous fibres, in particular hair, fur, hide and wool, faster are desirable as such compositions are very user convenient.

Further, it is desirable to be able to use a less amount of enzyme in the dyeing composition. This might make the dyeing process more economical. Further, the risk for creating airborne protein aerosols is reduced.

It has now surprisingly been found that it is possible to provide such improved dyeing compositions for keratinous fibres by using microbial laccases for oxidising the dye precursor(s).

Laccases (benzenediol:oxygen oxidoreductases) (E.C. class 1.10.3.2 according to Enzyme Nomenclature (1992) Academic Press, Inc.) are multi-copper containing enzymes that catalyse the oxidation of phenols. Laccase-mediated oxidation results in the production of aryloxy-radical intermediates from suitable phenolic substrates; the ultimate coupling of the intermediates so produced provides a combination of dimeric, oligomeric, and polymeric reaction products. Certain reaction products may be used to form dyes suitable for dyeing keratinous fibres, such as hair and wool (see below).

Firstly, the object of the invention is to provide a dyeing composition comprising
a) above 0 to 1 mg enzyme protein per ml dyeing composition of microbial laccase derived from a strain of the genus *Myceliophthora,*
b) one or more dye precursors,
c) optionally one or more modifiers.

In the second aspect the invention relates to the use of a dyeing composition of the invention for dyeing keratinous fibres, such as hair, fur, hide and wool.

The invention also related to a method for dying keratinous fibres comprising contacting the dyeing composition of the invention to the keratinous fibres in question under suitable conditions and for a period of time sufficient to permit oxidation of the dye precursor into a coloured compound.

The invention also relates to the use of a laccase for permanent dyeing of keratinous fibres wherein said laccase is a laccase that results in a ΔE*-value higher than the ΔE*-value resulting from a laccase derived from *Rhus* under corresponding dyeing conditions.

This means that when dyeing keratinous fibres with a dyeing composition of the present invention the ΔE*-value determined are higher than the ΔE*-value determined from corresponding keratinous fibres dyed under the same conditions using a dyeing composition comprising a laccase derived from *Rhus*.

The term "corresponding dyeing conditions" means under conditions where *e.g.* the enzyme concentration or enzyme activity, dyeing incubations time, dyeing incubation temperature, pH conditions, keratin fibre type (such as hair type) are the same, and further that the same dye precursor(s) and modifier(s) are used. In other words it defines conditions parallel to the specific dyeing conditions chosen. The dyeing conditions described below in the Examples may be chosen.

In the context of the present invention a "higher" ΔE* value defines that the total quantitative colour change is more than one ΔE* unit.

ΔE* is calculated from the values of the parameters a*, b* and L* determined *e.g.* on a Minolta CR200 Chroma Meter using the formula ΔE*= √(ΔL*²+Δa*²+Δb*²). The meaning of a*, b* and L* is explained below in the "Materials and Methods" section.

### BRIEF DESCRIPTION OF THE DRAWING

Figure 1 shows the dyeing effect of six different laccases. The six laccases are the *Polyporus pinsitus* laccase (rPp-laccase), *Myceliophthora thermophila* laccase (Mt-laccase wt.), *Myceliophthora thermophila* T1 variant laccase (Mt-laccase (var)), *Rhus vernicifera* laccase (Rvl-FXu-1), *Scytalidium thermophilum* laccase (rStL-FXu-1) and *Rhizoctonia solani* laccase (rRsL-3-FXu-1). o-aminophenol is used as the dye precursor and m-phenylene-diamine is used as a modifier.

Figure 2 shows the wash stability of the *Myceliophthora thermophila* T1 variant laccase (Mt-laccase (var)) and the *Polyporus pinsitus* laccase (rPp-laccase) as the oxidising agent.

Figure 3 shows the fastness (speed) of hair dyeing using the *Myceliophthora thermophila* T1 variant laccase (Mt-laccase (var)) and the *Polyporus pinsitus* laccase (rPp-laccase) as the oxidising agent.

Figure 4 shows the dose-response dyeing effect of *Myceliophthora thermophila* laccase, using from 0.0001 to 0.5 mg enzyme protein per ml dyeing composition. αα

### DETAILED DESCRIPTION OF THE INVENTION

The object of the present invention is to provide improved dyeing compositions for permanent dyeing of keratinous fibres, such as hair, fur, hide and wool, comprising an oxidation enzyme.

It has now surprisingly been found that it is possible to provide such improved dyeing compositions by using a microbial laccase derived from a strain of the genus *Myceliophthora* for oxidising the dye precursor(s).

### The Dyeing Composition

In the first aspect the present invention relates to a dyeing composition comprising
a) above 0 to 1 mg enzyme protein per ml dyeing composition of microbial laccase derived from a strain of the genus *Myceliophthora*,
b) one or more dye precursor, and
c) optionally one or more dye modifiers.

In a preferred embodiment of the invention the laccase may be present in the dyeing compositions in a concentration within the range from 0.0001 to 1 mg/ml, preferably 0.001 to 0.8 mg/ml, more preferred 0.002 to 0.5, even more preferred 0.003 to 0.2, especially 0.004 to 0.1 mg enzyme protein/ml dyeing composition.

When dyeing with a composition of the invention for permanent dyeing the ΔE*-value obtained is higher than that obtained when using a dyeing composition comprising a laccase derived from *Rhus* under corresponding dyeing conditions.

An example of a *Rhus* laccase is the laccase derived from the Japanese varnish tree *Rhus vernicifera* (Yoshida, (1883), J. Chem. Soc., 472). The *Rhus vernicifera* laccase is used in the Example 1 below.

The microbial laccase used according to the invention is derived from a strain of genus *Myceliophthora,* such as a strain of the species *Myceliophthora thermophila e.g.* the purified laccase described in WO 95/33836 (PCT/US95/06815) from Novo Nordisk, which is hereby incorporated by reference. SEQ ID NO 1 below shows a DNA sequence encoding a suitable laccase derivable from *Myceliophthora thermophila.*

*E*. *coli* JM101 containing the expression vector pRaMB5 comprising SEQ ID NO 1 has been deposited under the Budapest Treaty with the Agricultural Research Service Patent Culture Collection, Northern Regional Research Center, 1815 University Street, Peoria, Illinois, 61604. The vector have been given the Accession Number NRRL B-21261.

In addition, *Myceliophthora* laccases also encompass alternative forms of laccases which may be found in *M. thermophila* and as well as laccases which may be found in other fungi which are synonyms of fall within the definition of *M. thermophila* as described by Apinis (Nova Hedwigia 5, 57-78, 1963) and named *Sporotrichum thermophile.* Subsequent taxonomic revisions have placed this organism in the genus *Chysosporium* (Von Klopotek, A. Arche., (1974) Microbiol, 98, 365-369) and later *Myceliophthora* (Van Oorshot, Persoonia, (1977), 9, 401-408). A number of organisms known by other named also appear to belong to this species. These include *Sporotrichum cellulophilum* (US patent no. 4,106,989); *Thielavia thermophila* (Fergus and Sinden (1968), Can. J. Botany, 47, 1635-1637); *Chrysosporium fergussi* and *Corynascus thermophilus* (Von Klopotek, supra).

Also the use of laccase variants are contemplated according to the present invention.

An example of a laccase variant is the *Myceliophthora thermophila* T1 variant described in PCT/US96/14087 (Novo Nordisk).

T1 variants (or Type I variants) are modified blue copper oxidases, including laccases. T1 variants can for instance be constructed by site-directed mutagenesis and differ from the corresponding wild-type blue copper oxidases by at least one amino acid residue in the Type I (T1) copper site. These modifications generally result in altered pH profiles and/or specific activity relatively to the wild-type enzymes. This can be advantageous when using the enzyme in question in dyeing compositions.

More specific the *Myceliophthora thermophila* T1 laccase variant may comprise the sequence 509VSGGL511 or may be modified as to increase the negative charge in at least one segment of the T1 copper site.

The above mentioned microbial laccases may advantageously be used in permanent dyeing composition for keratinous fibres. Such compositions have a superior dyeing effect to corresponding compositions comprising *e.g.* the *Rhus vernicifera* laccase as shown in Example 1.

The *Myceliophthora thermophila* T1 variant laccase is more wash stabile and further dyes faster than the *Polyporus pinsitus* laccase which is proven in Example 2 and Example 3, respectively.

Example 4 shows that less *Myceliophthora* laccase activity (*i.e.* LACU/ml) is needed to obtain a suitable dyeing effect in comparison to the *Polyporus pinsitus* laccase.

Example 5 shows that for the *Myceliophthora thermophila* laccase the dyeing effect optimum is obtained around 0.005 mg enzyme protein per ml dyeing composition.

In the case of using a *Myceliophthora* laccase in a permanent dyeing composition it may advantageously be present in concentrations from above 0 to 1 mg/ml, preferably 0.0001 to 0.1 mg/ml, more preferably 0.0005 to 0.05 mg/ml, especially 0.001 to 0.01 mg enzyme protein per ml dyeing composition.

It is to be understood that the laccase may be produced either homologously, or heterologously in a host cell such as filamentous fungus, yeast or bacteria.

Examples of filamentous fungi host cells include strains of the species of *Trichoderma,* preferably a strain of *Trichoderma harzianum* or *Trichoderma reesei,* or a species of Fusarium, or a species of *Aspergillus,* most preferably *Aspergillus oryzae* or *Aspergillus niger,* or yeast cells, such as *e.g.* a strain of *Saccharomyces*, in particular *Saccharomyces cerevisiae*, *Saccharomyces kluyveri* or *Saccharomyces uvarum,* a strain of *Schizosaccharomyces* sp., such as *Schizosaccharomyces pombe,* a strain of *Hansenula* sp., *Pichia* sp., *Yarrowia* sp., such as *Yarrowia lipolytica,* or *Kluyveromyces* sp., such as *Kluyveromyces lactis,* or a bacteria, such as gram-positive bacteria such as strains of *Bacillus,* such as strains of *B. subtilis*, *B*. *Licheniformis*, *B*. *lentus*, *B*. *brevis*, *B*. *stearothermophilus*, *B*. *alkalophilus, B*. *amyloliquefaciens*, *B*. *coagulans*, *B*. *circulans*, *B. lautus*, *B*. *megaterium* or *B*. *thuringiensis,* or strains of *Streptomyces,* such as *S. lividans* or *S. murinus,* or gram-negative bacteria such as *Escherichia coli.*

To obtain dyeing of the keratinous fibres the dyeing composition of the invention comprises one or more dye precursors which is(are) converted into coloured compound(s) by an oxidation agent which according to the present invention is a microbial laccase.

Without being limited thereto the dye precursor(s) may be (an) aromatic compound(s) belonging to one of three major chemical families: the diamines, aminophenols (or aminonaphtols) and the phenols. Examples of isatin derivative dye precursors can be found in DE 4,314,317-A1. Further, a number of indole or indoline derivative dye precursors are disclosed in WO 94/00100. Said dye precursors mentioned in these documents are hereby incorporated herein by reference.

Examples of suitable dye precursors include compounds from the group comprising p-phenylene-diamine (PPD), p-toluylene-diamine (PTD), chloro-p-phenylene-diamine, p-aminophenol, o-aminophenol, 3,4-diaminotoluene, 2-methyl-1,4-diaminobenzene, 4-methyl-o-phenylenediamine, 2-methoxy-p-phenylenediamine, 2-chloro-1,4-diamino-benzene, 4-amino diphenylamine, 1-amino-4-β-methoxyethylamino-benzene, 1-amino-4-bis-(β-hydroxyethyl)-amonibenzene, 1-3-diamino-benzene, 2-methyl-1,3-diamino-benzene, 2,4-diaminotoluene, 2,6-diaminopyridine, 1-hydroxy-2-amino-benzene, 1-hydroxy-3-amino-benzene, 1-methyl-2-hydroxy-4-amino-benzene, 1-methyl-2-hydroxy-4-β-hydroxyethylamino-benzene, 1-hydroxy-4-amino-ebnzene, 1-hydroxy-4-methylamino-benzene, 1-methoxy-2,4-diamino-benzene, 1-ethoxy-2,3-diamino-benzene, 1-β-hy-droxyethyloxy-2,4-diamino-benzene, phenazines, such as 4,7-phenazinedicarboxylic acid, 2,7-phenazinedicarboxylic acid, 2-phenazinecarboxylic acid, 2,7-diaminophenazine, 2,8-diaminophenazine, 2,7-diamino-3,8-dimethoxyphenazine, 2,7-diamino-3-methoxyphenazine, 3-dimethyl 2,8-phenazinediamine, 2,2'-[(8-amino-7-methyl-2-phenazinyl)imino]bis-ethanol, 2,2'-[(8-amino-7-methoxy-2-phenazinyl)imino]bisethanol, 2,2'-[(8-amino-7-chloro-2-phenazinyl)imino]bis-ethanol, 2-[(8-amino-7-methyl-2-phenazinyl)amino]-ethanol, 2,2'-[(8-amino-2-phenazinyl)imino]bis-ethanol, 3-amino-7-(dimethylamino)-2,8-dimethyl-5-phenyl-chloride, 9-(diethylamino)- benzo [a] phenazine-1,5-diol, N-[8-(diethylamino)-2-phenazinyl]- methanesulfonamide, N-(8-methoxy-2-phenazinyl)- Methanesulfonamide, N,N,N',N'-tetramethyl-2,7-phenazinediamine, 3,7-dimethyl-2-phenazinamine, p-amino benzoic acids, such as p-amino benzoic acid ethyl, p-amino benzoic acid glycerid, p-amino benzoic acid isobutyl, p-dimethylamino benzoic acid amil, p-dimethylamino benzoic acid octyl, p-diethoxy amino benzoic amil, p- dipropoxy amino benzoic acid ethyl, acetylsalicylic acid, isatin derivatives, such as 2,3-diamino benzoic acid.

In an embodiment the laccase is used with the dye precursor directly to oxidise it into a coloured compound. The dye precursor may be used alone or in combination with other dye precursors.

It is believed that when using a diamine or an aminophenol as the dye precursor at least one of the intermediates in the copolymerisation must be an *ortho*- or *para*-diamine or aminophenol. Examples of such are found below and are also described in US patent no. 3,251,742 (Revlon), the contents of which are incorporated herein by reference.

Optionally dyeing compositions (especially hair dyeing compositions) of the invention also comprise a modifier (coupler) by which a number of colour tints can be obtained. In general modifiers are used in dyeing composition for hair as the hair colours resulting from hair dyeing compositions without modifier(s) are usually unacceptable for most people.

Modifiers are typically m-diamines, m-aminophenols, or polyphenols. Upon the optional addition of a modifier (coupler) it reacts with the dye precursor(s) in the presence of *e.g.* a laccase, converting the dye precursor(s) into a coloured compound.

Examples of modifiers (couplers) include m-phenylene-diamine, 2,4-diaminoanisole, 1-hydroxynaphthalene (α-naphthol), 1,4-dihydroxybenzene(hydroquinone), 1,5-dihydroxynapthalene, 1,2-dihydroxybenzene(pyrocatechol), 1,3-dihydroxybenzene (resorcinol), 1,3-dihydroxy-2-methylbenzene, 1,3-dihydroxy-4-chlorobenzene(4-chlororesorcinol), 1,2,3,trihydroxybenzene, 1,2,4-trihydroxybenzene, 1,2,4-trihydroxy-5-methylbenzene, 1,2,4-trihydroxytoluene.

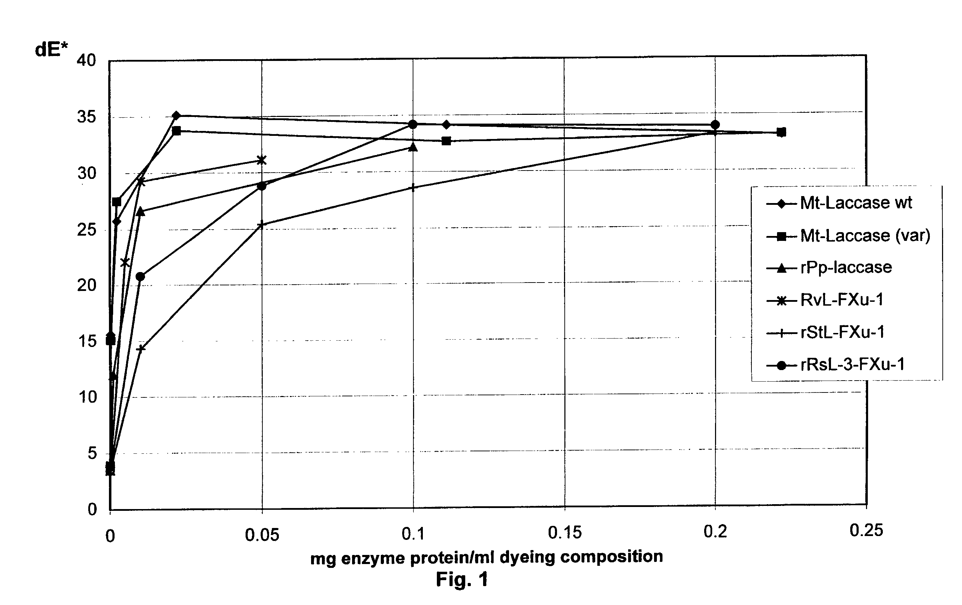

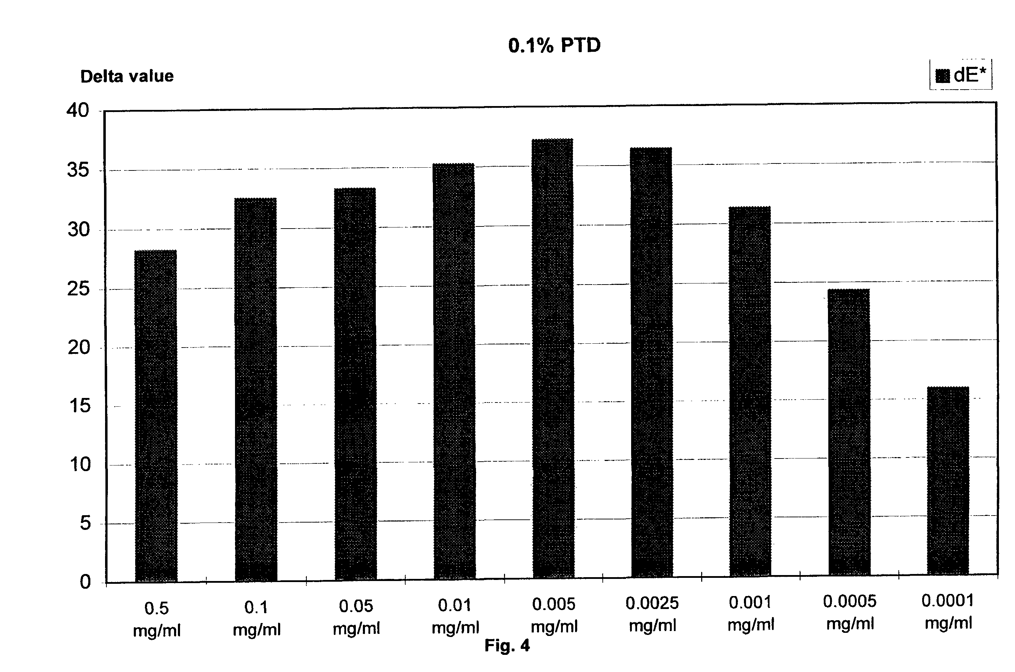

When using the dyeing compositions of the invention a reduced amount of enzyme (*i*.*e*. mg enzyme protein per ml dyeing composition) is needed to obtain the maximal dyeing effect (See Figure 1 and Figure 4), determined as the optimal ΔE*-value, in comparison to prior art dyeing compositions, such as dyeing compositions comprising a laccase derived from *Rhus*.

The amount of dye precursor(s) and other ingredients used in the composition of the invention are in accordance with usual commercial amounts.

According to the invention the product comprising the dyeing composition may be a one or a two compartment product. In the one compartment product the laccase, the dye precursor(s) and other ingredients are keep together in a stabilised solution or kept under stable conditions (*i.e.* the dye precursors are not oxidised by the laccase). In a two compartment product the laccase and the dye precursor(s) and other ingredients are keep in two containers keep apart. The contents of said containers are mixed immediately before use.

### USE

In the second aspect the invention relates to the use of the dyeing composition of the invention for permanent dyeing of keratinous fibres, in particular hair, fur, hide and wool.

When using a dying composition of the invention the ΔE*-value obtained is higher than that of a dyeing composition comprising a laccase derived from genus *Rhus* under corresponding dyeing conditions (see Figure 1).

### Method

In the third aspect the invention relates to a method for permanent dying of keratinous fibres comprising contacting a dyeing composition of the invention with the keratinous fibres in question under suitable conditions and for a period of time sufficient to permit oxidation of the dye precursor into a coloured compound.

The dyeing procedure may be carried out at room temperature, preferably around the optimal temperature of the enzyme, typically with from 10 to 60°C; at a pH in the range from 3 to 10, preferably 5 to 9, especially 6 to 8; for a period of time between 10 and 60 minutes, preferably 15 to 50 minutes, especially 20 to 40 minutes.

When using the method of the invention the ΔE*-value obtained is higher than that of corresponding methods where a laccase derived from a strain of the genus *Rhus* are used under the same dyeing conditions, in the presence or absence of at least one modifier, with at least one dye precursor, for a period of time, and under conditions sufficient to permit oxidation of the dye precursor used for oxidising the dye.

The method can be conducted with one or more dye precursors, either alone or in combination with one or more modifiers.

### MATERIALS AND METHODS

### Materials:

### Hair:

### 6" De Meo Virgin Natural White Hair (De Meo Brothers Inc. USA)

### Enzymes:

*Myceliophthora thermophila* laccase described in WO 95/33836 (PCT/US95/06815) from Novo Nordisk Biotech, Inc.
*Myceliophthora thermophila* T1 variant laccase described in US patent application 60/003,142 from Novo Nordisk Biotech, Inc.
*Polyporus pinsitus* laccase described in WO 96/00290 (PCT/US95/07536) from Novo Nordisk Biotech, Inc.
*Rhus vernicifera* laccase (Yoshida, J. Chem. Soc., 472 (1883)
*Rhizoctonia solani* laccase described in WO 95/07988 from Novo Nordisk Biotech, Inc.
*Scytalidium thermophilum* laccase described in WO 95/33837 (PCT/US95/06816) from Novo Nordisk Biotech, Inc.

### Deposit of Biological Material

The following biological material has been deposited on the 25 May 1994 under the terms of the Budapest Treaty with the Agricultural Research Service Patent Culture Collection, Northern Regional Research Center, 1815 University Street, Peoria, Illinois, 61604 and given the following accession number.

| Deposit | Accession Number |
|---|---|
| *E. coli* JM101 containing pRaMB5 | NRRL B-21261 |

### Dye precursors:

0.1 % w/w p-phenylene-diamine in 0.1 M K-phosphate buffer, pH 7.0. (pPD)
0.1 % w/w p-toluylene-diamine in 0.1 M K-phosphate buffer, pH 7.0.
0.1 % w/w chloro-p-phenylenediamine in 0.1 M K-phosphate buffer, pH 7.0.
0.1 % w/w p-aminophenol in 0.1 M K-phosphate buffer, pH 7.0.
0.1 % w/w o-aminophenol in 0.1 M K-phosphate buffer, pH 7.0.
0.1 % w/w 3,4-diaminotoluene in 0.1 M K-phosphate, buffer pH 7.0.

### Modifiers:

0.1 % w/w m-phenylenediamine in 0.1 M K-phosphate buffer, pH 7.0.
0.1 % w/w 2,4-diaminoanisole in 0,1 M K-phosphate buffer, pH 7.0.
0.1 % w/w alpha-naphthol in 0.1 M K-phosphate buffer, pH 7.0.
0.1 % w/w hydroquinone in 0.1 M K-phosphate buffer, pH 7.0.
0.1 % w/w pyrocatechol in 0.1 M K-phosphate buffer, pH 7.0.
0.1% w/w resorcinol in 0.1 M K-phosphate buffer, pH 7.0.
0.1 % w/w 4-chlororesorcinol in 0.1 M K-phosphate buffer, pH 7.0.

The dye precursor is combined with one of the above indicated modifiers so that the final concentration in the dyeing solution is 0.1 % w/w with respect to precursor and 0.1 % w/w with respect to modifier.

### Other solutions:

### Commercial shampoo

### Equipment:

### Minolta CR200 Chroma Meter

### Determination of Laccase Activity (LACU)

Laccase activity is determined from the oxidation of syringaldazin under aerobic conditions. The violet colour produced is photometered at 530 nm. The analytical conditions are 19 mM syringaldazin, 23.2 mM acetate buffer, pH 5.5, 30°C, 1 min. Reaction time.

1 laccase unit (LACU) is the amount of enzyme that catalyses the conversion of 1.0 micromole syringaldazin per minute at these conditions.

### Assessment of the hair colour

The quantitative colour of the hair tresses is determined on a Minolta CR200 Chroma Meter by the use the parameters L* ("0"=black and "100"=white), a* ("-"=green and "+"=red) and b* ("-" blue and "+" yellow).

ΔL*, Δa* and Δb* are the delta values of L*, a* and b* respectively compared to L*, a* and b* of untreated hair (*e.g.* ΔL* = L*ₛₐₘₚₗₑ - L*_{untreated hair}).

ΔE* is calculated as ΔE*=√(ΔL*²+Δa*²+Δb*²) and is an expression for the total quantitative colour change.

### EXAMPLES

### Example 1

### Dyeing effect

The dyeing effect of different laccases were tested and compared under the same conditions using 0.1% w/w o-aminophenol (dye precursor) and 0.1% w/w m-phenylene-diamine (modifier).

The laccases tested were
a *Polyporus pinsitus* laccase,
a *Myceliophthora thermophila* laccase
a *Myceliophthora thermophila* T1 laccase variant,
a *Rhus vernicifera* laccase
a *Rhizoctonia solani* laccase
a *Scytalidium thermophila* laccase

### Hair dyeing

1 gram white De Meo hair tresses were used.

4 ml dye precursor solution (including modifier) was mixed with 1 ml laccase on a Whirley mixer, applied to the hair tresses and incubated at 30°C for 60 minutes.

The hair tresses were then rinsed with running water, washed with shampoo, rinsed with water, combed, and air dried.

a*, b* and L* were determined on the Chroma Meter and ΔE* was then calculated.

Hair tress samples treated without enzyme were used as a blind.

The result of the test is displayed in figure 1.

### Example 2

### Wash stability

Tresses of white De Meo hair (1 gram) were used for testing of the wash stability of hair dyed using the *Myceliophthora thermophila* T-variant laccase and the *Polyporus pinsitus* laccase.

Oxidative hair dyeing was carried out as described in Example 1, except that p-phenylene-diamine (pPD) were used as the dye precursor and no modifiers were used.

### Hair wash

The dyed hair tresses were wetted and washed for 15 seconds with 50 ml of commercial shampoo, and rinsed with water for 1 minute and air dried. The hair tresses were washed up to 18 times.

Then a*, b* and L* were determined on the Chroma Meter and ΔE* values were then calculated.

Hair tress samples treated without enzymes were used as blinds.

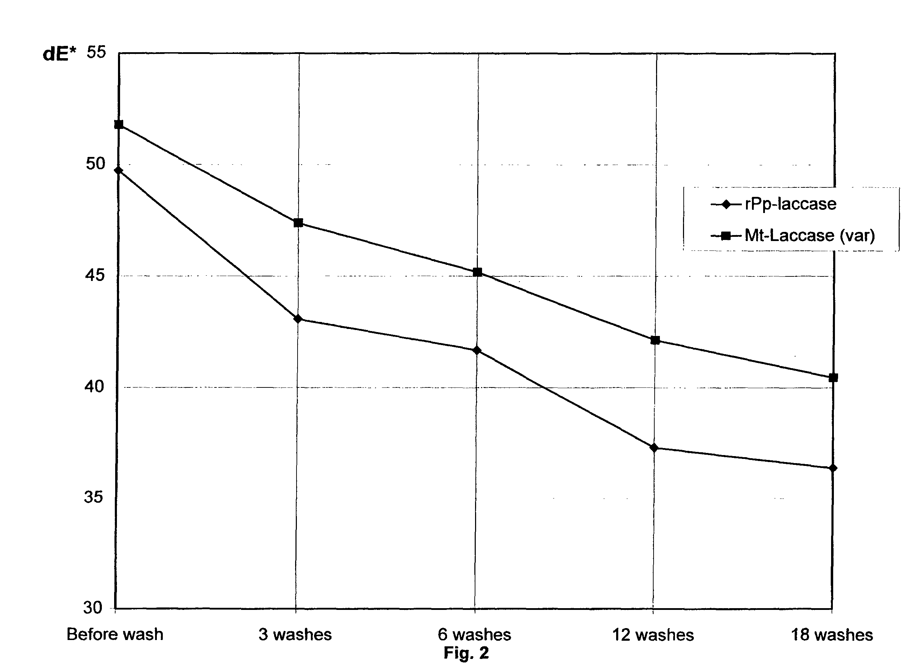

The result of the test is displayed in figure 2.

### Example 3

### Fastness of hair dyeing

Tresses of white De Meo hair (1 gram) were used for testing fastness (speed) of hair dyeing using the *Myceliophthora thermophila* T1 variant laccase and the *Polyporus pinsitus* laccase.

p-phenylene-diamine (pPD) was used as the dye precursor and no modifiers were used.

4 ml dye precursor solution was mixed with 1 ml laccase on a Whirley mixer, applied to the hair tresses and incubated at 30°C for 10, 20, 30, 40, 50 and 60 minutes, respectively.

The hair tresses were then rinsed with running water, washed with shampoo, rinsed with running water, combed, and air dried.

a*, b* and L* were determined on the Chroma Meter for each incubation time and the ΔE*-values were then calculated.

Hair tress samples treated without enzymes for 60 minutes were used as blinds.

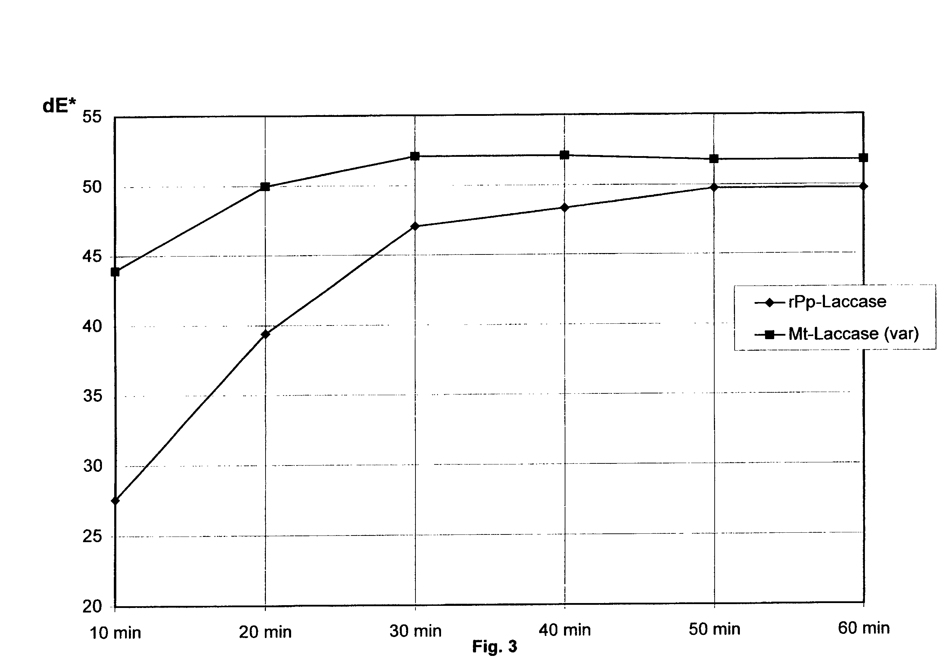

The result of the test is displayed in figure 3.

### Example 4

### Dyeing effect of Myceliophthora thermophila T1 variant laccase

The dyeing effect of *Myceliophthora thermophila* T1 variant laccase were compared with the *Polyporus pinsitus* laccase using 0.1% w/w p-phenylene-diamine, 0.1% w/w p-touylene-diamine, 0.1% w/w chloro-p-phenylene-diamine, 0.1% w/w p-aminophenol, 0.1% w/w o-aminophenol and 0.1% w/w 3,4 diaminotoluene, respectively, as dye precursors.

The *Polyporus pinsitus* laccase were applied in a concentration of 10 LACU/ml while the *Myceliophthora thermophila* T1 variant laccase was applied in a concentration of only 1 LACU/ml.

1 gram white De Meo hair tresses were used.

4 ml dye precursor solution was mixed with 1 ml laccase on a Whirley mixer, applied to the hair tresses and incubated at 30°C for 60 minutes.

The hair tresses were then rinsed with running water, washed with shampoo, rinsed with running water, combed, and air dried.

The a*, b* and L* were determined on the Chroma Meter and the ΔE* values were then calculated.

Hair tress samples treated without enzyme were used as blinds.

The result of the test is displayed in Table 1.

**Table 1**

| Sample | *Polyporus pinsitus* laccase ΔE* | *Myceliophthora thermophila* T1 variant laccase ΔE* |
|---|---|---|
| p-phenylene-diamine blind | 9.7 | 10.9 |
| p-phenylene-diamine + laccase | 52.7 | 52.9 |
| p-toluylene-diamine blind | 16.1 | 18.6 |
| p-toluylene-diamine + laccase | 39.1 | 38.2 |
| chloro-p-phenylene-diamine blind | 2.6 | 4.0 |
| chloro-p-phenylene-diamine + laccase | 40.5 | 39.2 |
| p-aminophenol blind | 6.2 | 7.0 |
| p-aminophenol + laccase | 32.4 | 28.1 |
| o-amonophenol blind | 5.6 | 6.4 |
| o-amonophenol + laccase | 22.9 | 22.0 |
| 3,4-diaminotoluene blind | 3.4 | 2.6 |
| 3,4-diaminotoluene + laccase | 36.5 | 42.2 |

As can be seen from Table 1 compositions comprising the *Myceliophthora thermophila* T1 laccase variant dyes the hair as good as the *Polyporus pinsitus* laccase even though concentration of the *Polyporus pinsitus* laccase is 10 time higher.

### Example 5

### Dose-response dyeing effect of M. thermophila laccase

The dyeing effect of *M*. *thermophila* laccase were tested using concentration between 0.0001 to 0.5 mg enzyme protein per ml dyeing composition of laccase. 0.1% w/w p-toluylene-diamine (PTD) was used as the dye precursor.

The same dyeing procedure as described in Example 1 was used.

The result of the tests are displayed in Figure 4.

## Claims

1. A dyeing composition comprising
a) above 0 to 1 mg enzyme protein per ml dyeing composition of microbial laccase derived from a strain of the genus *Myceliophthora,*
b) one or more dye precursor, and
c) optionally one or more dye modifiers.

2. The dyeing composition according claims 1, wherein the laccase is presents in a concentration of from 0.0001 to 1 mg/ml, preferably 0.001 to 0.8 mg/ml, more preferred 0.002 to 0.5 mg/ml, even more preferred 0.003 to 0.2 mg/ml, especially 0.004 to 0.1 mg enzyme protein/ml dyeing composition.

3. The dyeing composition according to claims 1 and 2, wherein said microbial laccase is of filamentous fungus origin.

4. The dyeing composition according to claims 1 anti 2, wherein the *Myceliophthora* laccase is derived from a strain of species *Myceliophthora thermophila,* such as *Myceliophthora thermophila* NRRL B 21261, or variants thereof, such as the T1 variant.

5. The dyeing composition according to claim 4, wherein the laccase is encoding by the sequence shown in SEQ ID NO 1.

6. The dyeing composition according to claims 4 and 5, wherein the laccase is present in a concentration of from above 0 to 1 mg/ml, preferably 0.0001 to 0.1 mg/ml, more preferably 0.0005 to 0.05 mg/ml, especially 0.001 to 0.01 mg enzyme protein/ml dyeing composition.

7. The dyeing composition according to any of claims 1 to 6, comprising a dye precursor selected from the group comprising p-phenylene-diamine (pPD), p-toluylene-diamine (pTD), chloro-p-phenylenediamine, p-aminophenol, o-aminophenol, 3,4-diaminotoluene, 2-methyl-1,4-diaminobenzene, 4-methyl-o-phenylenediamine, 2-methoxy-p-phenylenediamine, 2-chloro-1,4-diamino-benzene, 4-amino diphenylamine, 1-amino-4-β-methoxyethylamino-benzene, 1-amino-4-bis-(β-hydroxyethyl)-amonibenzene, 1-3-diaminobenzene, 2-methyl-1,3-diamino-benzene, 2,4-diaminotoluene, 2,6-diaminopyridine, 1-hydroxy-2-amino-benzene, 1-hydroxy-3-aminobenzene, 1-methyl-2-hydroxy-4-amino-benzene, 1-methyl-2-hydroxy-4-β-hydroxyethylamino-benzene, 1-hydroxy-4-amino-benzene, 1-hydroxy-4-methylamino-benzene, 1-methoxy-2,4-diamino-benzene, 1-ethoxy-2,3-diamino-benzene, 1-β-hydroxyethyloxy-2,4-diaminobenzene, phenazines, such as 4,7-phenazinedicarboxylic acid, 2,7-phenazinedicarboxylic acid, 2-phenazinecarboxylic acid, 2,7-diaminophenazine, 2,8-diaminophenazine, 2,7-diamino-3,8-dimethoxyphenazine, 2,7-diamino 3-methoxyphenazine, 3-dimethyl 2,8-phenazinediamine, 2,2'-[(8-amino-7-methyl-2-phenazinyl) imino] bis-ethanol, 2,2'-[(8-amino-7-methoxy-2-phenazinyl) imino] bis-ethanol, 2,2'-[(8-amino-7-chloro-2-phenazinyl)imino]bis-ethanol, 2-[(8-amino-7-methyl-2-phenazinyl)amino]-ethanol, 2,2'-[(8-amino-2-phenazinyl) imino]-bis-ethanol, 3-amino-7-(dimethylamino)-2,8-dimethyl-5-phenylchloride, 9-(diethylamino)- benzo[a]phenazine-1,5-diol, N-[8-(diethylamino)-2-phenazinyl]-methanesulfonamide, N-(8-methoxy-2-phenazinyl)-methanesulfonamide, N,N,N',N'-tetramethyl-2,7-phenazinediamine, 3,7-dimethyl-2-phenazinamine, p-amino benzoic acids, such as p-amino benzoic acid ethyl, p-amino benzoic acid glycerid, p-amino benzoic acid isobutyl, p-dimethylamino benzoic acid amil, p-dimethylamino benzoic acid octyl, p-diethoxy amino benzoic amil, p-dipropoxy amino benzoic acid ethyl, acetylsalicylic acid, isatin derivatives, such as 2,3-diamino benzoic acid.

8. The dyeing composition according to any of claims 1 to 7, comprising a dye modifier selected from the group comprising m-phenylene-diamine, 2,4-diaminoanisole, 1-hydroxynaphthalene(α-naphthol), 1,4-dihydroxybenzene (hydroquinone), 1,5-dihydroxynapthalene, 1,2-dihydroxybenzene (pyrocatechol), 1,3-dihydroxybenzene (resorcinol), 1,3-dihydroxy-2-methylbenzene, 1,3-dihydroxy-4-chlorobenzene (4-chlororesorcinol), 1,2,3,trihydroxybenzene, 1,2,4-trihydroxybenzene, 1,2,4-trihydroxy-5-methylbenzene, 1,2,4-trihydroxytoluene.

9. Use of the composition according to claim 1 to 8 for permanent dyeing of keratinous fibres, such as hair, fur, hide or wool.

10. A method for dying keratinous fibres comprising contacting a dyeing composition according to claims 1 to 8 to the keratinous fibres under suitable conditions and for a period of time sufficient to permit oxidation of the dye precursor into a coloured compound.

11. The method according to claim 10, wherein the dyeing procedure is carried out at a pH in the range from 3 to 10, preferably 5 to 9, especially 6 to 8.

12. The method according to claims 10 and 11, wherein the procedure is carried out for a period of time between 10 and 60 minutes, preferably 15 to 50 minutes, especially 20 to 40 minutes.

## Patentansprüche

1. Färbezusammensetzung, umfassend
(a) mehr als 0 bis 1 mg Enzymprotein pro ml Färbezusammensetzung von mikrobieller Laccase, die von einem Stamm der Gattung *Myceliophthora* stammt,
(b) einen oder mehrere Farbstoffvorläufer und
(c) gegebenenfalls einen oder mehrere Farbstoffmodifikator(en).

2. Färbezusammensetzung nach Anspruch 1, worin die Laccase in einer Konzentration von 0,0001 bis 1 mg/ml, vorzugsweise 0,001 bis 0,8 mg/ml, bevorzugter 0.002 bis 0,5 mg/ml, noch bevorzugter 0,003 bis 0,2 mg/ml, insbesondere 0,004 bis 0,1 mg Enzymprotein/ml Färbezusammensetzung, vorliegt.

3. Färbezusammensetzung nach den Ansprüchen 1 und 2, worin die mikrobielle Laccase von einem filamentösen Pilz stammt.

4. Färbezusammensetzung nach den Ansprüchen 1 und 2, worin die *Myceliophthora-*Laccase von einem Stamm der Spezies *Myceliophthora thermophila*, z.B. *Myceliophtora thermophila* NRRL B 21261, oder Varianten davon, z.B. der T1-Variante, stammt.

5. Färbezusammensetzung nach Anspruch 4, worin die Laccase von der in SEQ-ID-NR. 1 dargestellten Sequenz kodiert wird.

6. Färbezusammensetzung nach den Ansprüchen 4 und 5, worin die Laccase in einer Konzentration von mehr als 0 bis 1mg/ml, vorzugsweise 0,0001 bis 0,1 mg/ml, bevorzugter 0,0005 bis 0,05 mg/ml, insbesondere 0,001 bis 0,01 mg Enzymprotein/ml Färbezusammensetzung, vorliegt.

7. Färbezusammensetzung nach irgendeinem der Ansprüche 1 bis 6, umfassend einen Farbstoffvorläufer, der aus der Gruppe, umfassend p-Phenylendiamin (pPD), p-Toluylendiamin (pTD), Chlor-p-phenylendiamin, p-Aminophenol, o-Aminophenol, 3,4-Diaminotoluol, 2-Methyl-1,4-diaminobenzol, 4-Methyl-o-phenylendiamin, 2-Methoxy-p-phenylendiamin, 2-Chlor-1,4-diaminobenzol, 4-Aminodiphenylamin, 1-Amino-4-β-methoxyethylaminobenzol, 1-Amino-4-bis-(β-hydroxyethyl)-aminobenzol, 1,3-Diaminobenzol, 2-Methyl-1,3-diaminobenzol, 2,4-Diarninotoluol, 2,6-Diaminopyridin, 1-Hydroxy-2-Aminobenzol, 1-Hydroxy-3-aminobenzol, 1-Methyl-2-hydroxy-4-aminobenzol, 1-Methyl-2-hydroxy-4-β-hydroxyethylaminobenzol, 1-Hydroxy-4-aminobenzol, 1-Hydroxy-4-methylaminobenzol, 1-Methoxy-2,4-diaminobenzol, 1-Ethoxy-2,3-diaminobenzol, 1-β-Hydroxethyloxy-2,4-diaminobenzol, Phenazine, z.B. 4,7-Phenazindicarbonsäure, 2,7-Phenazindicarbonsäure, 2-Phenazincarbonsäure, 2,7-Diaminophenazin, 2,8-Diaminophenazin, 2,7-Diamino-3,8-dimethoxyphenazin, 2,7-Diamino-3-methoxyphenazin, 3-Dimethyl-2,8-phenazindiamin, 2,2'-[(8-Amino-7-methyl-2-phenazinyl)imino]bisethanol, 2,2'-[(8-Amino-7-methoxy-2-phenazinyl)imino]bisethanol, 2,2'-[(8-Amino-7-Chlor-2-phenazinyl)imino]bisethanol, 2-[(8-Amino-7-methyl-2-phenazinyl)amino]ethanol, 2,2'-[(8-Amino-2-phenazinyl)imino]-bisethanol. 3-Amino-7-(dimethylamino)-2,8-dimethyl-5-phenylchlorid, 9-(Diethylamino)benzo[a]phenazin-1,5-diol, N-[8-(Diethylamino)-2-phenazinyl]methansulfonamid, N-[8-methoxy-2-phenazinyl]methansulfonamid, N, N, N', N'-Tetramethyl-2,7-phenazindiamin, 3,7-Dimethyl-2-phenazinamin, p-Aminobenzoesäuren, z.B. p-Aminobenzoesäureethyl, p-Aminobenzoesäureglycerid, p-Aminobenzoesäureisobutyl, p-Dimethylaminobenzoesäureamyl, p-Dimethylaminobenzoesäureoctyl, p-Diethoxyaminobenzoesäureamyl, p-Dipropoxyaminobenzoesäureethyl, Acetylsalicylsäure, Isatin-Derivate, z.B. 2,3-Diaminobenzoesäure, ausgewählt ist.

8. Färbezusammensetzung nach irgendeinem der Ansprüche 1 bis 7, umfassend einen Farbstoffmodifikator, der aus der Gruppe, umfassend m-Phenylendiamin, 2,4-Diaminoanisol, 1-Hydroxynaphthalin (α-Naphthol), 1,4-Dihydroxybenzol (Hydrochinon), 1,5-Dihydroxynaphthalin, 1,2-Dihydroxybenzol (Brenzcatechin), 1,3-Dihydroxybenzol (Resorcin), 1,3-Dihydroxy-2-methylbenzol, 1,3-Dihydroxy-4-Chlorbenzol (4-Chlorresorcin), 1,2,3-Trihydroxybenzol, 1,2,4-Trihydroxybenzol, 1,2,4-Trihydroxy-5-methylbenzol, 1,2,4-Trihydroxytoluol, ausgewählt ist.

9. Verwendung der Zusammensetzung nach den Ansprüchen 1 bis 8 zur dauerhaften Färbung von keratinhaltigen Fasern wie z.B. Haar, Pelz, Haut oder Wolle.

10. Verfahren zur Färbung von keratinhaltigen Fasern, umfassend das Kontaktieren einer Färbezusammensetzung nach den Ansprüchen 1 bis 8 mit den keratinhaltigen Fasern unter geeigneten Bedingungen und für eine ausreichende Zeitspanne, um die Oxidation des Farbstoffvorläufers zu einer gefärbten Verbindung zu erlauben.

11. Verfahren nach Anspruch 10, worin die Färbeprozedur bei einem pH-Wert im Bereich von 3 bis 10, vorzugsweise 5 bis 9, insbesondere 6 bis 8, durchgeführt wird.

12. Verfahren nach den Ansprüchen 10 und 11, worin die Prozedur für eine Zeitspanne zwischen 10 und 60 Minuten, vorzugsweise 15 bis 50 Minuten, insbesondere 20 bis 40 Minuten, durchgeführt wird.

## Revendications

1. Composition colorante comportant :
a) plus de 0 à 1 mg de protéine enzymatique par ml de composition colorante de laccase microbienne dérivée d'une souche du genre *Myceliophthora*,
b) un ou plusieurs précurseurs de colorant, et
c) de manière facultative, un ou plusieurs modificateurs de colorant.

2. Composition colorante selon la revendication 1, dans laquelle la laccase est présente selon une concentration allant de 0,0001 à 1 mg/ml, de préférence de 0,001 à 0,8 mg/ml, de manière plus préférée de 0,002 à 0,5 mg/ml, de manière encore plus préférée de 0,003 à 0,2 mg/ml, en particulier de 0,004 à 0,1 mg de protéine enzymatique/ml de composition colorante.

3. Composition colorante selon les revendications 1 et 2, dans laquelle ladite laccase microbienne a pour origine un champignon filamenteux.

4. Composition colorante selon les revendications 1 et 2, dans laquelle la laccase du genre *Myceliophthora* est dérivée d'une souche de l'espèce *Myceliophthora thermophila,* telle que *Myceliophthora thermophila* NRRL B 21261, ou des variants de celle-ci, tels que le variant T1.

5. Composition colorante selon la revendication 4, dans laquelle la laccase est codée par la séquence représentée dans la SEQ ID N° 1.

6. Composition colorante selon les revendications 4 et 5, dans laquelle la laccase est présente selon une concentration allant de plus de 0 à 1 mg/ml, de préférence de 0,0001 à 0,1 mg/ml, de manière plus préférée de 0,0005 à 0,05 mg/ml, en particulier de 0,001 à 0,01 mg de protéine enzymatique/ml de composition colorante.

7. Composition colorante selon l'une quelconque des revendications 1 à 6, comportant un précurseur de colorant sélectionné parmi le groupe constitué des éléments suivants : p-phénylène-diamine (pPD), p-toluène-diamine (pTD), chloro-p-phénylènediamine, p-aminophénol, o-aminophénol, 3,4-diamino-toluène, 2-méthyle-1,4- diaminobenzène, 4-méthyle-o-phénylènediamine, 2-métoxy-p-phénylènediamine, 2-chloro-1,4-diamino-benzène, 4-amino diphénylamine, 1-amino-4-β-méthoxyéthylamino-benzène, 1-amino-4-bis-(β-hydroxyéthyle)-amonibenzène, 1-3- diamino-benzène, 2-méthyle-1,3-diamino-benzène, 2,4-diamino-toluène, 2,6-diaminopyridine, 1-hydroxy-2-amino-benzène, 1-hydroxy-3-amino-benzène, 1-méthyle-2-hydroxy-4-amino-benzène, 1-méthyle-2-hydroxy-4-β-hydroxyéthylamino- benzène, 1-hydroxy-4-amino-benzène, 1-hydroxy-4-méthylamino-benzène, 1-méthoxy-2,4-diamino-benzène, 1-éthoxy-2,3-diamino-benzène, 1-β-hydroxyéthyloxy-2,4-diamino-benzène, des phénazines, tels que l'acide 4,7-phénazinedicarboxylique, l'acide 2,7-phénazinedicarboxylique, l'acide 2-phénazinedicarboxylique, 2,7-diaminophénazine, 2,8-diaminophénazine, 2,7-diamino-3,8-diméthoxyphénazine, 2,7-diamino-3-méthoxyphénazine, 3-diméthyle-2,8-phénazinediamine, 2,2'-[(8-amino-7-méthyle-2-phénazinyle) imino]bis-éthanol, 2,2'-[(8-amino-7-méthoxy-2-phénazinyle) imino]bis-éthanol, 2,2'-[(8-amino-7-chloro-2-phénazinyle) imino]bis-éthanol, 2-[(8-amino-7-méthyle-2-phénazinyle) amino]-éthanol, 2,2'-[(8-amino-2-phénazinyle) imino]bis-éthanol, chlorure de 3-amino-7-(diméthylamino)-2,8-diméthyle-5-phényle, 9-(diéthylamino)-benzo[a]phénazine-1,5-diol, N-[8-(diéthylamino)-2-phénazinyle]-méthanesulfonamide, N-(8-méthoxy-2-phénazinyle)-méthanesulfonamide, N, N, N', N'-tétraméthyle-2,7-phénazinediamine, 3,7-diméthyle-2-phénazinamine, des acides p-amino benzoïque, tels que l'éthyle d'acide p-amino benzoïque, glycéride d'acide p-amino benzoïque, isobutyle d'acide p-amino benzoïque, amile d'acide p-diméthylamino benzoïque, octyle d'acide p-diméthylamino benzoïque, amile d'acide p-diéthoxy amino benzoïque, éthyle d'acide p-dipropoxy amino benzoïque, acide acétylsalicylique, des dérivés d'isatine, tels que l'acide 2,3-diamino benzoïque.

8. Composition colorante selon l'une quelconque des revendications 1 à 7, comportant un modificateur de colorant sélectionné parmi le groupe comportant les éléments suivants : m-phénylène-diamine, 2,4-diaminoanisole, 1-hydroxynaphtalène (α-naphtol), 1,4-dihydroxybenzène(hydroquinone), 1,5-dihydroxynaphtalène, 1,2-dihydroxybenzène(pyrocatéchol), 1,3-dihydroxybenzène (resorcinol), 1,3-dihydroxy-2- méthylbenzène, 1,3-dihydroxy-4-chlorobenzène (4-chlororesorcinol), 1,2,3,trihydroxybenzène, 1,2,4-trihydroxybenzène, 1,2,4-trihydroxy-5-méthyle-benzène, 1,2,4,-trihydroxytoluène.

9. Utilisation de la composition selon les revendications 1 à 8, pour une coloration permanente de fibres de kératine, telles que des cheveux, de la fourrure, du cuir ou de la laine.

10. Procédé pour colorer des fibres de kératine comportant la mise en contact d'une composition colorante selon les revendications 1 et 8 et des fibres de kératine sous des conditions adaptées et pendant une période de temps suffisante pour permettre l'oxydation du précurseur de colorant en un composé coloré.

11. Procédé selon la revendication 10, dans lequel le procédé de coloration est effectué à un pH situé dans la plage allant de 3 à 10, de préférence de 5 à 9, en particulier de 6 à 8.

12. Procédé selon les revendications 10 et 11, dans lequel le procédé est effectué pendant une période de temps comprise entre 10 et 60 minutes, de préférence 10 et 50 minutes, en particulier entre 20 et 40 minutes.
